# EUROPEAN PATENT SPECIFICATION

(11) **EP 0 837 066 B1**
(45) Date of publication and mention of the grant of the patent: **03.09.2003**
(21) Application number: 96917662.7
(22) Date of filing: 13.06.1996
(51) Int. Cl.: C07D 499/46

(54) **IMPROVED PROCESS FOR PRODUCING PENICILLIN G PHENYL ESTER**
VERFAHREN ZUR HERSTELLUNG VON PENICILLING-PHENYLESTER
PROCEDE AMELIORE DE PRODUCTION DE PHENYL-ESTER DE PENICILLINE G

(30) Priority: 14.06.1995 JP 14752895; 07.03.1996 JP 8078296
(43) Date of publication of application: 22.04.1998
(73) Proprietor: TAKEDA SCHERING-PLOUGH ANIMAL HEALTH K.K., Osaka 541-0046 (JP)
(72) Inventor: TAKAHASHI, Satoru, Nishinomiya-shi, Hyogo 663 (JP); YAMAMURA, Atsushi, Osaka-shi, Osaka 532 (JP); HAYASHIDA, Hisashi, Daito-shi, Osaka 574 (JP)
(74) Representative: Gille Hrabal Struck Neidlein Prop Roos
(86) International application number: JP9601604
(87) International publication number: WO97000259

(56) References cited:
- EP-A- 0 453 924
- WO-A-93/08196
- DD-A- 150 393
- JP-A- 4 108 793
- ABRAHAM,D.J. ET AL.: "Design, Synthesis and Testing of antisickling agnts. 2. Proline Derivatives Designed for the Donor Site* " J.MED.CHEM., vol. 26, no. 4, 1983, WASHINGTON, pages 549-554, XP002074733

## Description

### TECHNICAL FIELD

This invention relates to a novel process for producing penicillin G phenyl esters. More particularly, this invention relates to a process for producing penicillin G phenyl esters advantageously on a commercial scale by way of intermediates which are novel compounds.

### BACKGROUND ART

The objective compound (I) of this invention shows a very favorable absorption characteristic following oral administration when used as a therapeutic agent for enterococcicosis in fish, for instance, and the following route of synthesis is known for its production [PCT/JP92/01327)

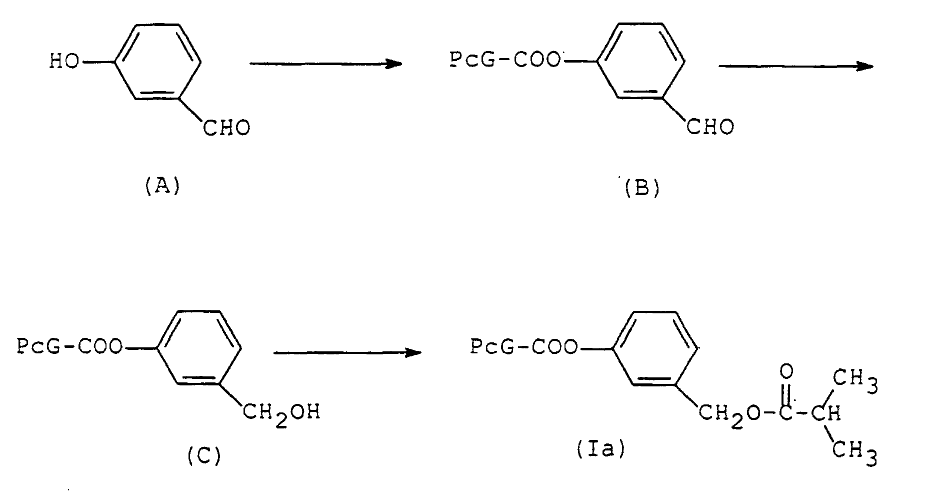

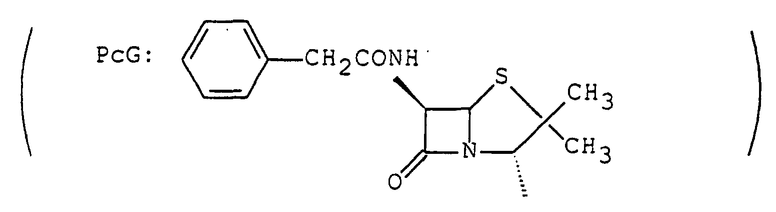

However, this process involves handling of the above compounds (B) and (C) each having the unstable penicillin skeleton and allergenic to man and, therefore, unsafe to production workers.

### DISCLOSURE OF THE INVENTION

The inventors of this invention explored for a safe and commercially advantageous production technology and discovered a safe and industrially useful process for producing penicillin G phenyl esters via the following intermediates (II) and (IIa) which are novel compounds.

Compared with the intermediates (B) and (C) mentioned above in connection with the conventional process, the intermediate (II) described hereinafter, particularly the following intermediate (IIa): 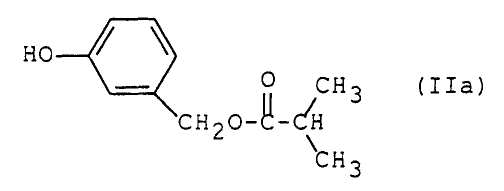 has a small molecular mass offering the additional advantage that, process-wise, a smaller-capacity reactor suffices for the intermediate reaction step.

The objective compound (I) of the invention can be represented by the formula:

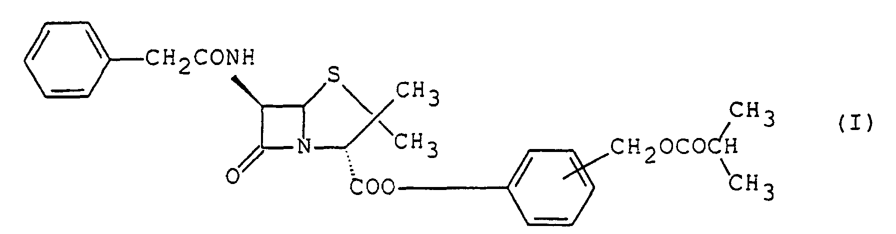

In accordance with this invention, the objective compound (I) can be produced by acylating a compound of the formula: 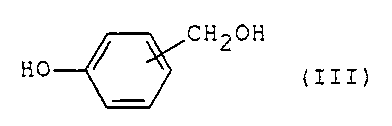 or a salt thereof to provide a compound of the formula: 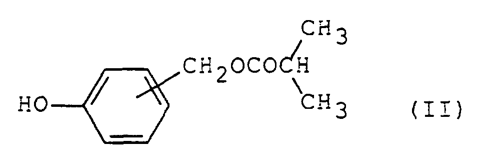 or a salt thereof and reacting it further with a compound of the formula: 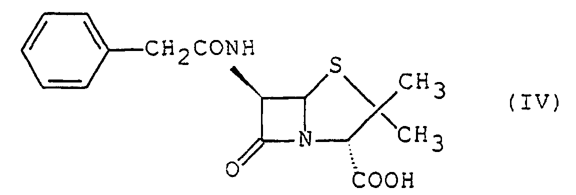 or a salt thereof to provide a compound of the formula: 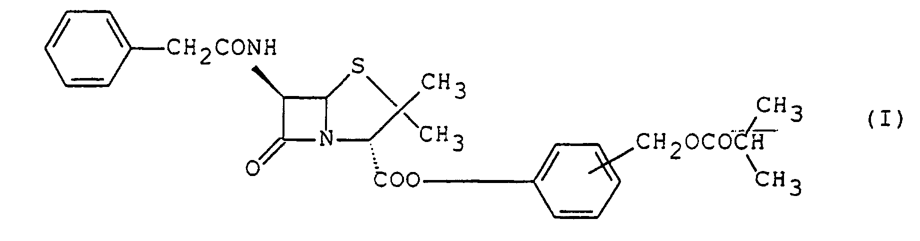 or a salt thereof.

In accordance with this invention, the objective compound (I) can be produced by the following processes. 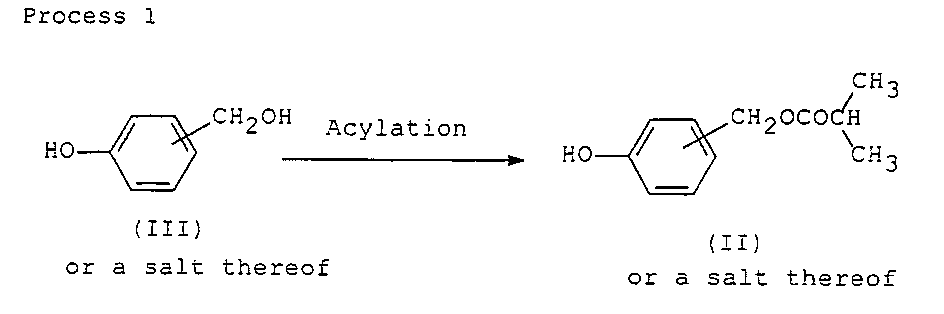 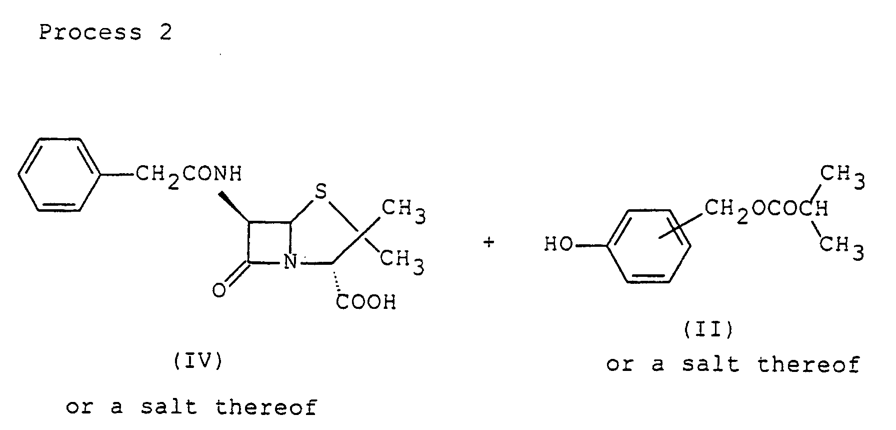 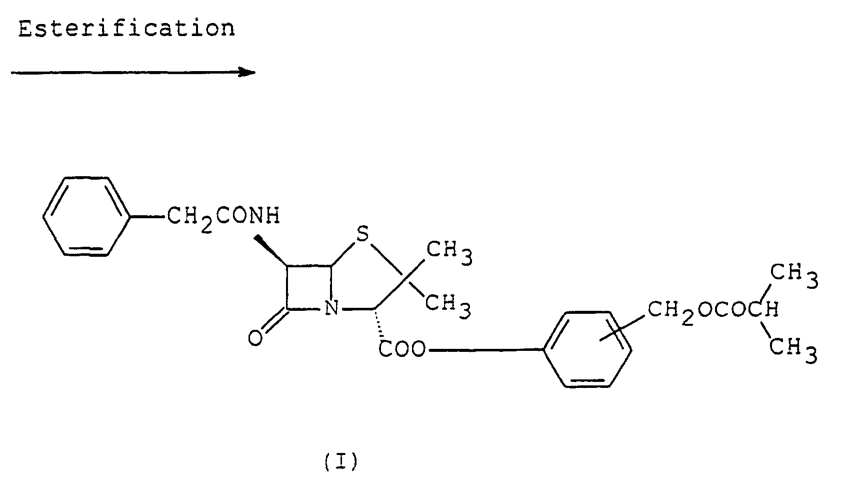 or a salt thereof

The pharmaceutically acceptable salt of the objective compound (I) is preferably a nontoxic salt of the common kind, which includes various acid addition salts. Thus, inorganic acid addition salts (e.g. hydrochloride, hydrobromide, sulfate, phosphate, etc.), organic carboxylic or sulfonic acid addition salts (e.g. formate, acetate, trifluoroacetate, oxalate, maleate, fumarate, tartrate, methanesulfonate, benzenesulfonate, p-toluenesulfonate, etc.), and salts with acidic amino acids (e.g. aspartate, glutamate, etc.) can be mentioned.

### BEST MODE FOR CARRYING OUT THE INVENTION

The processes for producing the objective compound (I) are now described in detail.

### Process 1

Compound (II) or a salt thereof can be produced by subjecting compound (III) or a salt thereof to selective acylation of its alcoholic hydroxyl group.

The preferred salt of compound (II) and of compound (III) includes salts with inorganic bases, such as alkali metal salts (e.g. sodium salt, potassium salt, cesium salt, etc.), alkaline earth metal salts (e.g calcium salt, magnesium salt, etc.), and ammonium salts; salts with organic bases, such as organic amine salts (e.g. trimethylamine salt, triethylamine salt, pyridine salt, picoline salt, ethanolamine salt, triethanolamine salt, dicyclohexylamine salt, N,N'-dibenzylethylenediamine salt, etc.).

The acylating agent which can be used for this acylation reaction includes the compound of the formula: 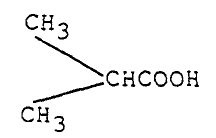 or a salt thereof, or a reactive derivative thereof. The preferred reactive derivative is an acid halide, although an acid anhydride may also be employed.

The preferred acid halide includes the acid chloride and acid bromide.

The preferred "acid anhydride" includes mixed anhydrides with alkanoic acids (e.g. the mixed anhydride with acetic acid) and those with alkenoic acids (e.g. the mixed anhydride with 2-butenoic acid) as well.

As it is the case with compound (III), a compound having a phenolic and an alcoholic hydroxyl group within its molecule undergoes preferential acylation of the alcoholic hydroxyl group under acidic or neutral conditions. This selectivity of acylation is accentuated when the reaction is conducted in the presence of an acid acceptor (e.g. N,N-dimethylacetamide, N,N-diethylacetamide, N,N-dimethylpropionamide, N-methyl-2-pyrrolidone, N-methyl-acetanilide, tetramethylurea, tetraethylurea, etc.). Particularly preferred is N-methyl-2-pyrrolidone.

This reaction is generally conducted in the common solvent such as acetone, dioxane, tetrahydrofuran, diethyl ether, diisopropyl ether, acetonitrile, chloroform, methylene chloride, ethylene chloride, ethyl acetate, N,N-dimethylformamide, toluene, etc. or a mixture of such solvents, although the reaction may be optionally carried out in any other organic solvent that does not interfere with the reaction.

There is no particular limitation on the reaction temperature but the reaction is generally carried out under cooling, at room temperature, or under warming.

### Process 2

The objective compound (I) or salt mentioned above can be produced by reacting compound (IV) or a salt thereof with compound (II) or a salt thereof.

The preferred salt of compound (IV) includes those corresponding to the above-mentioned salts of compound (II).

This reaction is generally carried out by the conventional esterification procedure, e.g. in the presence of a condensing agent which is routinely used, such as carbodiimides (e.g. N,N'-dicyclohexylcarbodiimide, N-cyclohexyl-N'-morpholinoethylcarbodiimide, N-cyclohexyl-N'-(4-diethylaminocyclohexyl)carbodiimide, N,N'-diethylcarbodiimide, N,N'-diisopropylcarbodiimide, N-ethyl-N'-(3-dimethylaminopropyl)carbodiimide, etc.), triazoles (e.g. 1-(p-chlorobenzenesulfonyloxy)-6-chloro-1H-benzotriazole, N-hydroxybenzotriazole, etc.), halopyridinium salts (e.g. 2-chloro-1-methylpyridinium iodide etc.), cyanuric chloride-pyridine complex, thionyl chloride-dimethylformamide (Vilsmeier's reagent), methanesulfonyl chloride, etc. or the mixed acid anhydride method. Preferred among the above-mentioned condensing agents are cyanuric chloride-pyridine complex, thionyl chloride-dimethylformamide (Vilsmeier's reagent), and N,N'-dicyclohexylcarbodiimide.

This reaction is generally carried out in the presence of an inorganic or organic base such as alkali metal hydroxides (e.g. sodium hydroxide, potassium hydroxide, etc.), alkali metal carbonates (e.g. sodium carbonate, potassium carbonate, etc.), alkali metal hydrogen carbonates (e.g. sodium hydrogen carbonate, potassium hydrogen carbonate, etc.), tri(lower)alkylamines (e.g. trimethylamine, triethylamine, etc.), pyridine and its derivatives (e.g. picoline, lutidine, 4-dimethylaminopyridine, etc.), N-(lower)alkylmorpholine, and N,N-di(lower)alkylbenzylamine, among other bases.

This reaction is generally conducted in the common solvent such as dioxane, tetrahydrofuran, diethyl ether, diisopropyl ether, acetonitrile, chloroform, methylene chloride, ethylene chloride, ethyl acetate, N,N-dimethylformamide, etc. or using any desired organic solvent that does not interfere with the reaction.

The condensing agent or the base, which is liquid, can be used as the solvent as well.

There is no particular limitation on the reaction temperature but the reaction is generally carried out under cooling, at room temperature, or under heating.

The compound obtained by the above process can be isolated and purified by routine procedures such as, for example, extraction, precipitation, fractional crystallization, recrystallization, distillation under reduced pressure, and chromatography.

The present invention provides a production technology for penicillin G phenyl ester (I) which is superior to the prior art technology in both safety and amenability to commercial-scale production.

The following experiment comparing the process of the invention with the prior art process (PCT/JP92/01327) is demonstrative of the superiority of the process of the invention in terms of product yield.

### Prior Art Process

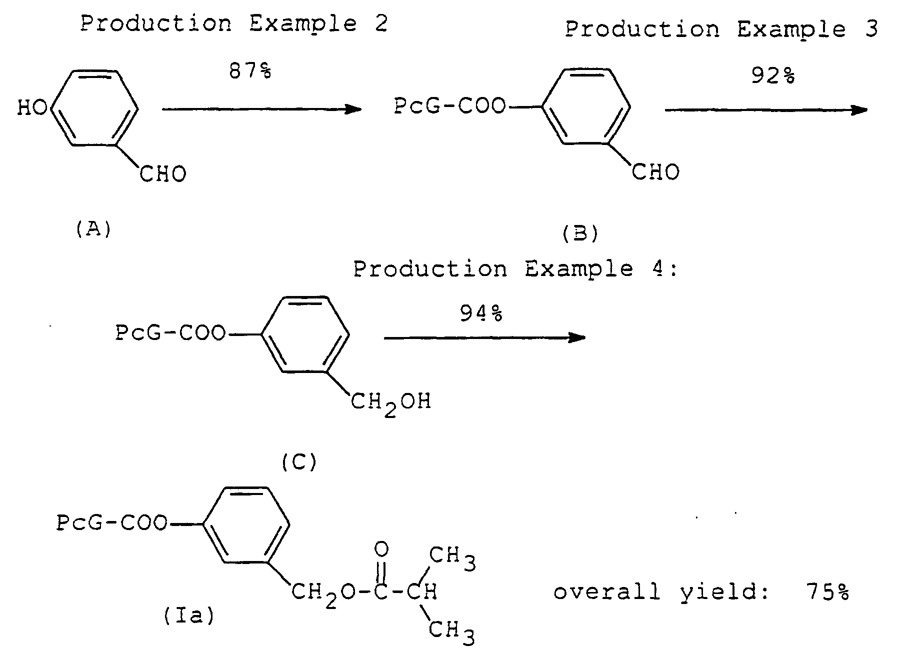

### Process of the invention

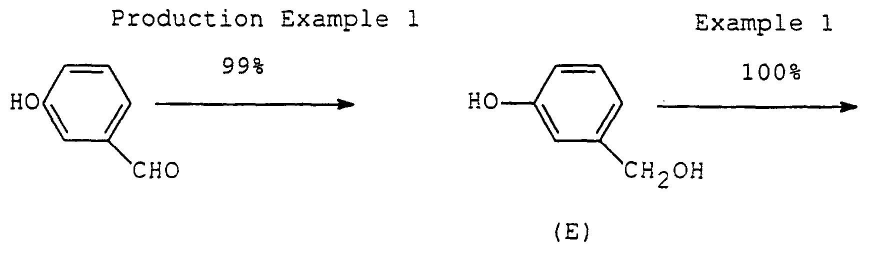

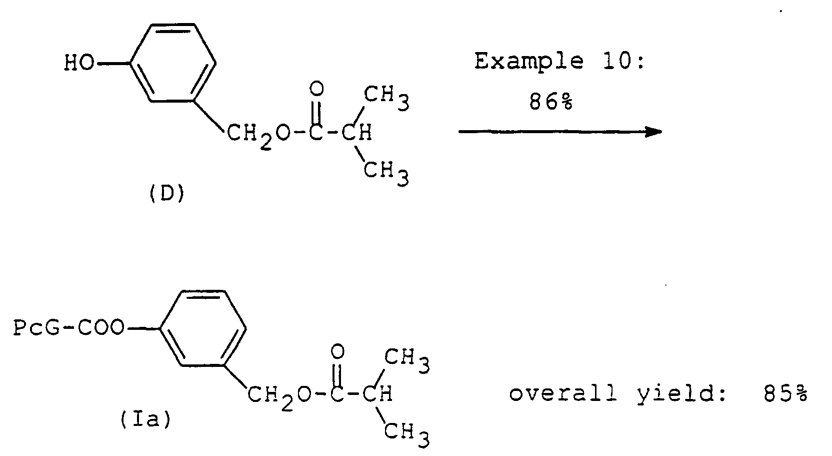

In addition to said higher yield, the process of this invention has the following advantages over the prior art process. Thus, the intermediate (B) used in the prior art process does not crystallize but is only available as an oil and its purification has to depend on silica gel column chromatography which is not suited for commercial production. On the other hand, the intermediate (D) for use in the process of this invention, although it is oily, too, can be easily purified by distillation under reduced pressure (b.p. 150°C/0.4 Torr).

Thus, the process of this invention is superior to the prior art process in yield, operability, and economics (the intermediates (E) and (D) in the process of this invention are low molecular weight compounds as compared with the intermediates (B) and (C) in the prior art process and, therefore, the volume of the reaction system is smaller on an equimolar reaction basis).

### Production Example 1

3-Hydroxybenzaldehyde (20.1 g) is suspended in water (41 ml). To this suspension is added sodium borohydride (1.65 g) in a nitrogen atmosphere at 20-30°C, and the mixture is stirred for 30 minutes. At a temperature not exceeding 20°C, concentrated hydrochloric acid (about 2.5 ml) is added gradually to adjust the mixture to pH 7. To this mixture, ethyl acetate (41 ml) and sodium chloride (11.5 g) are added for extraction. The organic layer is separated, dried over anhydrous magnesium sulfate (10 g), and concentrated under reduced pressure to 30 ml. To this residue is added 205 ml of n-heptane for crystallization. The resulting crystals are recovered by filtration and dried in vacuo to provide white crystals of 3-hydroxybenzyl alcohol (20.2 g; yield 99%).

### Production Example 2

Penicillin G potassium (10.0 g) is suspended in pyridine (50 ml), and in a nitrogen atmosphere at 20 ∼ -10°C, a solution of cyanuric chloride (5.71 g) in tetrahydrofuran (40 ml) is added dropwise. Then, at the same temperature, 3-hydroxybenzaldehyde (2.76 g) is added dropwise and washed in with tetrahydrofuran (10 ml). The mixture is stirred at -5° - 0°C for 2.5 hours, at the end of which time ethyl acetate (100 ml) and water (150 ml) are added. The mixture is allowed to stand and the organic layer is separated and washed twice with an ice-cooled mixture of 2N-hydrochloric acid (100 ml) and sodium chloride (20 g). This solution is further washed once with an ice-cooled mixture of water (100 ml), sodium hydrogen carbonate (5.0 g) and sodium chloride (8.0 g) and twice with saturated aqueous solution of sodium chloride (50 ml). The organic layer is dried over anhydrous magnesium sulfate, decolorized with activated carbon, and concentrated under reduced pressure to recover 14.49 g of residue. Assay of this residue by liquid chromatography shows that it contains 8.59 g (yield 87%) of penicillin G 3-formylphenyl ester.

### Production Example 3

To a solution of penicillin G 3-formylphenyl ester (content 8.6 g) in ethyl acetate (16.25 g) is added 40 ml of methanol and after the mixture is cooled to -20°C ∼10°C, sodium borohydride (209 mg) is added with stirring. Then, at -10°C ∼ -0°C, water (50 ml) is added dropwise for crystallization. The resulting crystals are recovered by filtration and dried in vacuo to provide penicillin G 3-hydroxymethylphenyl ester as white crystals (7.94 g, yield 92%).

### Production Example 4

A mixture of penicillin G 3-hydroxymethylphenol (7.68 g), 4-dimethylaminopyridine (52 mg), and dimethylformamide (5 ml) is cooled to a temperature not over 10°C and isobutyric anhydride (2.96 g) is added. This mixture is stirred at 25°C for 2 hours, after which isopropyl alcohol (27 ml) is added. The reaction mixture is then filtered and isopropyl alcohol (50 ml) is further added to the filtrate. After addition of seed crystals, the mixture is stirred. Then, water (120 ml) is added dropwise for crystallization. The resulting crystals are recovered by filtration and dried in vacuo to provide penicillin G 3-isobutyryloxymethylphenyl ester (8.14 g, 94%).

### EXAMPLES

### Example 1

In tetrahydrofuran (20 ml) is dissolved 3-hydroxybenzyl alcohol (8.0 g), and isobutyryl chloride (8.9 g) is then added dropwise to the above solution in such a manner that the internal temperature will not exceed 30°C. To this reaction mixture is added saturated aqueous sodium chloride solution (40 ml) to stop the reaction. The organic layer is then separated and washed twice each with saturated aqueous sodium chloride solution (40 ml) and saturated aqueous sodium hydrogen carbonate solution (40 ml) and once with saturated aqueous sodium chloride solution (40 ml). The organic layer is dried over anhydrous magnesium sulfate and concentrated under reduced pressure to provide 3-isobutyryloxymethylphenol (12.8 g) as oil.
IR (Nujol): 3325, 1697, 1587 cm⁻¹.
NMR (CDCl₃, δ): 1.47 (6H, d, J=7.0 Hz), 2.61 (1H, hep, J=7.0 Hz), 5.07 (2H, s), 5.40 (1H, s), 6.7-6.9 (3H, m), 7.22 (1H, t, J=7.7 Hz).

### Example 2

A mixture of 3-hydroxybenzyl alcohol (25.1 g), N-methyl-2-pyrrolidone (32.0 g) and dichloromethane (150 ml) is cooled to 10°C. To this cooled mixture is added isobutyryl chloride (25.7 g) dropwise over 15 minutes. After completion of dropwise addition, the mixture is further stirred at room temperature for 30 minutes. This reaction mixture is adjusted to pH 6 with a dilute solution of sodium hydroxide and extracted with dichloromethane.
The organic layer is separated, washed 3-5 times with water and saturated aqueous solution of sodium hydrogen carbonate, and concentrated under reduced pressure to recover about 40 g of oily residue. This residue is purified by silica gel column chromatography (eluent: n-hexane-ethyl acetate = 4:1∼3:1) to provide 3-isobutyryloxymethylphenol (32.6 g) as oil.

### Example 3

A mixture of penicillin G potassium (60.0 g) and pyridine (29.7 g) is added dropwise with stirring in a nitrogen atmosphere at -10° ∼ -20°C. Then, at the same temperature, 3-isobutyryloxymethylphenol (26.0 g) is added dropwise. After completion of dropwise addition, the mixture is further stirred at -20° ∼ 0°C for 1 hour. This reaction mixture is diluted with water (400 ml) and ethyl acetate (350 ml) and then adjusted to pH3 with dilute HCl for extraction. The organic layer is separated, washed with saturated aqueous solution of sodium hydrogen carbonate twice, dried over anhydrous sodium sulfate, and concentrated. To the oily residue is added diisopropyl ether and the mixture is stirred for crystallization. The crude crystals are collected by filtration and dissolved in methanol (500 ml) under warming. After treatment with activated carbon, the solution is concentrated and caused to crystallize again from diisopropyl ether. This crystal crop is harvested by filtration to provide penicillin G 3-isobutyryloxymethylphenyl ester (52.6 g) as white crystals.
NMR (CDCl₃, δ): 1.19 (6H, d, J=7.0 Hz), 1.53 (3H, s), 1.63 (3H, s), 2.61 (1H, hep, J=7.0 Hz), 3.66 (2H, s), 4.60 (1H, s), 5.11 (2H, s), 5.56 (1H, d, J=4.2 Hz), 5.70 (1H, dd, J=4.2, 9.01 Hz), 6.10 (1H, d, J=9.0 Hz), 7.0-7.5 (9H, m).

### Example 4

While a mixture of penicillin G potassium (60.0 g) and pyridine (300 ml) is maintained at -10° ∼ -20°C in a nitrogen atmosphere, a solution of cyanuric chloride (29.7 g) in dichloromethane (180 ml) is added dropwise with stirring. Then, at the same temperature, 3-isobutyryloxymethylphenol (26.0 g) is added dropwise. After completion of dropwise addition, the mixture is further stirred at -20° ∼ 0°C for 1 hour. This reaction mixture is diluted with water (400 ml) and ethyl acetate (350 ml) and then adjusted to pH3 with dilute HCl for extraction. The organic layer is separated, washed with saturated aqueous solution of sodium hydrogen carbonate twice, dried over anhydrous sodium sulfate, and concentrated. To the resulting oil is added diisopropyl ether (400 ml) and the mixture is stirred for crystallization. The crude crystals are collected by filtration and dissolved in methanol (500 ml) under warming, and after treatment with activated carbon (5 g), the solution is concentrated and caused to crystallize again from diisopropyl ether (250 ml). This crystal crop is harvested by filtration to provide penicillin G 3-isobutyryloxymethylphenyl ester (52.6 g) as white crystals.
IR (Nujol): 3370, 1780, 1770, 1760, 1730, 1686, 1516 cm⁻¹
NMR (CDCl₃, δ): 1.19 (6H, d, J=7.0 Hz), 1.53 (3H, s), 1.63 (3H, s), 2.61 (1H, hep, J=7.0 Hz), 3.66 (2H, s), 4.60 (1H, s), 5.11 (2H, s), 5.56 (1H, d, J=4.2 Hz), 5.70 (1H, dd, J=4.2, 9.01 Hz), 6.10 (1H, d, J=9.0 Hz), 7.0-7.5 (9H, m).

### Example 5

In dichloromethane (40 ml) are suspended penicillin G potassium (6.2 g), 3-isobutyryloxymethylphenol (2.5 g) and 2-chloro-1-methylpyridinium iodide (4.3 g), and while this suspension is stirred at room temperature, triethylamine (1.7 g) is added dropwise in such a manner that the internal temperature will not exceed 35°C. After completion of dropwise addition, the mixture is further stirred at room temperature for 30 minutes. This reaction mixture is concentrated under reduced pressure and the residue is distributed using water (60 ml) and ethyl acetate (60 ml). The aqueous layer is further extracted with ethyl acetate (30 ml). The organic layers are combined, washed with water (30 ml) once, dried over anhydrous sodium sulfate, and concentrated under reduced pressure. The resulting oil is purified by silica gel column chromatography (silica gel 100 g; eluent: ethyl acetate-n-hexane = 1:2) to provide penicillin G 3-isobutyryloxymethylphenyl ester (3.43 g) as white crystals. The physical constants of this compound are in agreement with those of the compound synthesized in Example 4.

### Example 6

In tetrahydrofuran (40 ml) are suspended penicillin G potassium (6.1 g), 3-isobutyryloxymethylphenol (2.5 g) and N,N'-dicyclohexylcarbodiimide (3.5 g), followed by addition of 4-dimethylaminopyridine (70 mg). While this mixture is stirred at room temperature, 4N-HCl/ethyl acetate (4 ml) is added dropwise in such a manner that the internal temperature will remain at 25°-35°C. After completion of dropwise addition, the mixture is stirred at room temperature for 30 minutes and, then, suction-filtered.
The filtrate is concentrated under reduced pressure and the residue is distributed using water (70 ml) and ethyl acetate (70 ml). The ethyl acetate layer is separated, dried over anhydrous sodium sulfate, and concentrated under reduced pressure. The residual oil is purified by silica gel column chromatography (silica gel 70 g; eluent: ethyl acetate-n-hexane = 1:2) to provide penicillin G 3-isobutyryloxymethylphenyl ester (5.22 g) as white crystals. The physical constants of this compound are in agreement with those of the compound synthesized in Example 4.

### Example 7

A suspension of penicillin G potassium salt (4.60 g) in methylene chloride (18 ml) is cooled to a temperature not exceeding -5°C and N-methylmorpholine (1.2 ml) and methanesulfonyl chloride (1.4 ml) are added in that order at -5°C with constant stirring. The mixture is further stirred at a temperature not over 0°C for 90 minutes. Then, 3-isobutyryloxymethylphenol (2.21 g) is added dropwise at 0° ∼ -5°C. The drip funnel is washed with methylene chloride (4 ml) and the washes are added to the reaction mixture. The mixture is then stirred at 0° ∼ -5°C for 2 hours. This reaction mixture is diluted with water (14 ml) and the organic layer is separated, washed serially with 5% citric acid, 25% aqueous NaCl, 10% aqueous NaHCO₃, 25% aqueous NaCl, and water (14 ml each), dried over anhydrous magnesium sulfate, and finally concentrated to dryness under reduced pressure. The resulting crude crystals are dissolved in ethyl acetate (9 ml), and n-heptane (46 ml) is added dropwise for crystallization. The crystal crop is harvested by filtration and dried in vacuo to provide penicillin G 3-isobutyryloxymethylphenyl ester as white crystals (2.63 g). The physical constants of the compound thus obtained are in greement with those of the compound synthesized in Example 4.

### Example 8

To dimethylformamide (30 ml) is added thionyl chloride (7.35 g) at a temperature not exceeding 0°C and the mixture is stirred well. The stirring is further continued at a temperature not over 0°C, and penicillin G potassium (21.10 g), 3-isobutyryloxymethylphenol (10.94 g), and pyridine (9.76 g) are added in the order mentioned. The mixture is then stirred at -10° ∼ 0°C for 30 minutes, after which ethyl acetate (100 ml) and 20% aqueous NaCl solution (100 ml) are serially added. The organic layer was separated and washed with 20% NaCl, 10% citric acid-20% NaCl (1:1), 5% NaHCO₃-20% NaCl (1:1), and 20% NaCl (100 ml each) in the order mentioned. The organic layer is separated and concentrated under reduced pressure and the residual oil is dissolved in isopropyl alcohol (100 ml). While this solution is stirred at 20°-25°C, seed crystals are added and water (150 ml) is then added dropwise for crystallization. The resulting crystals are collected by filtration and dried in vacuo to provide penicillin G 3-isobutyryloxymethylphenyl ester (24.3 g). The physical constants of the compound thus obtained are in agreement with those of the compound synthesized in Example 4.

### Example 9

A mixture of cyanuric chloride (3.70 g) and ethyl acetate (54 ml) is cooled to -10° ∼ -5°C. Then, pyridine (7.94 g) is added dropwise with stirring at the same temperature. After thorough stirring, dimethylformamide (27 ml), penicillin G potassium salt (6.90 g) and 3-isobutyryloxymethylphenol (3.0 g) are added in that order and the mixture is stirred at -10° ∼ -5°C for 5 hours. Then, at room temperature, the reaction mixture is washed twice with 5% citric acid and 10% aqueous NaCl (60 ml each) and once with 2.5% aqueous NaHCO₃, 10% aqueous NaCl, and water (60 ml each). The organic layer is concentrated to dryness under reduced pressure and the residue is dissolved in isopropyl alcohol (30 ml) at 20°-25°C. After addition of seed crystals, the mixture is stirred for 30 minutes, after which water (45 ml) is added dropwise for crystallization. The crystals are collected by filtration and dried to provide penicillin G 3-isobutyryloxymethylphenyl ester (6.95 g) as white crystals. The physical constants of this compound are in agreement with those of the compound synthesized in Example 4.

### Example 10

A mixture of penicillin G potassium (2.0 kg) and pyridine (10 ℓ) is cooled to -15°C in a nitrogen atmosphere. To this mixture is added a solution of cyanuric chloride (1.1 kg) in tetrahydrofuran (8 ℓ) with stirring at an internal temperature of -10° ∼ -20°C. Then, at the same, temperature, 3-isobutyryloxymethylphenol (0.89 kg) is added dropwise. After completion of dropwise addition, the mixture is stirred at -5° ∼ 0°C for 2.5 hours. This reaction mixture is diluted with ethyl acetate (20 ℓ) and washed once with water (30 ℓ), twice with a cooled dilute HCl/aqueous NaCl solution (22 ℓ) twice with 10% aqueous NaHCO₃ solution (10 ℓ), and once with saturated aqueous NaCl solution (10 ℓ) in the order mentioned. The organic layer is dried (over MgSO₄) and filtered with the aid of activated carbon. The filtrate is concentrated under reduced pressure to about 4l and N-hexane (22 ℓ) is added to the residue for crystallization. The crystals are collected by filtration to provide penicillin G 3-isobutyryloxymethylphenyl ester (2.0 kg) as white crystals. The physical constants of this compound are in agreement with those of the compound synthesized in Example 4.

## Claims

1. A process for producing a penicillin G phenyl ester of the formula: 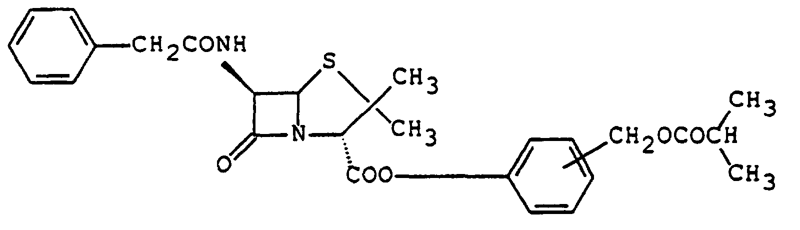 or a salt thereof which comprises reacting a compound of the formula: 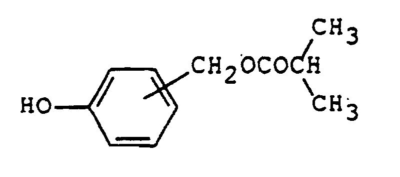 or a salt thereof with a compound of the formula: 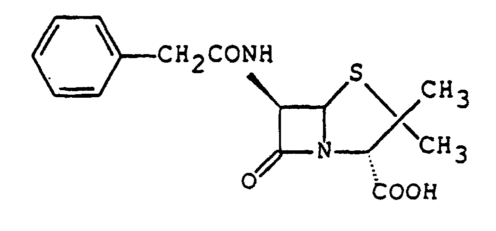 or a salt thereof.

2. The process according to Claim 1 wherein the reaction is conducted in the presence of a condensing agent.

3. A process for producing a penicillin G phenyl ester of the formula: 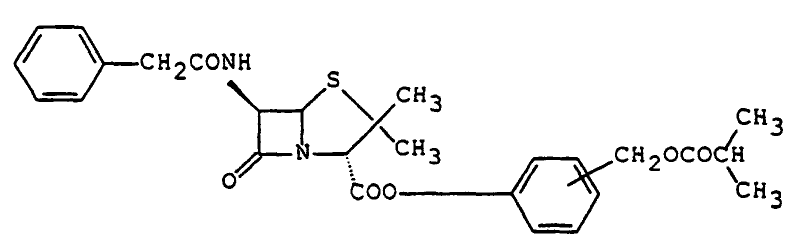 or a salt thereof which comprises acylating a compound of the formula: 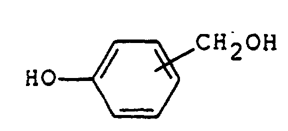 or a salt thereof to provide a compound of the formula: 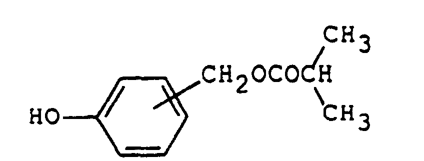 or a salt thereof and reacting the same with a compound of the formula: 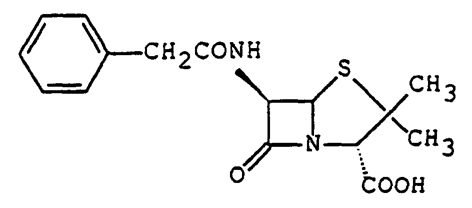 or a salt thereof.

4. A process for producing a penicillin G phenyl ester of the formula: 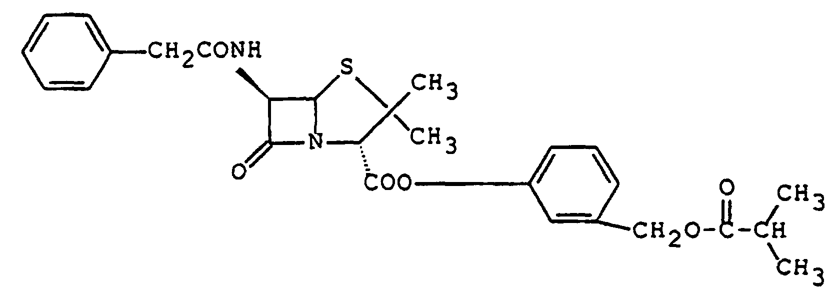 or a salt thereof which comprises acylating a compound of the formula: 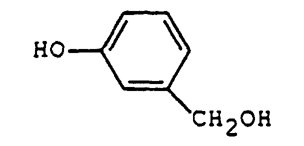 or a salt thereof to provide a compound of the formula: 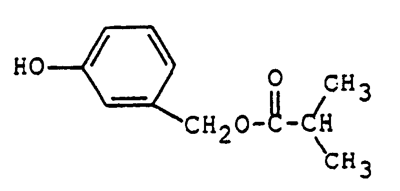 or a salt thereof and reacting the same with a compound of the formula: 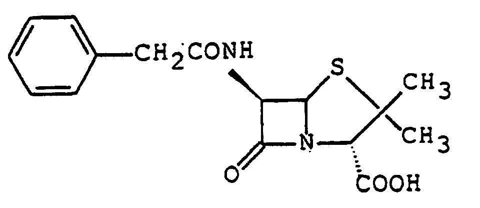 or a salt thereof.

5. A compound of the formula: 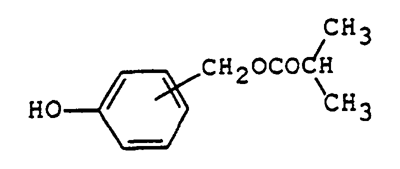 or a salt thereof.

6. A compound of the formula: 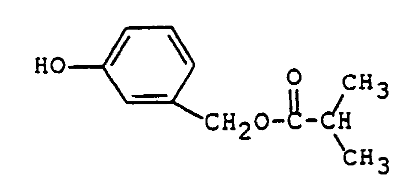 or a salt thereof.

## Patentansprüche

1. Verfahren zur Herstellung eines Penicillin-G-Phenylesters der Formel 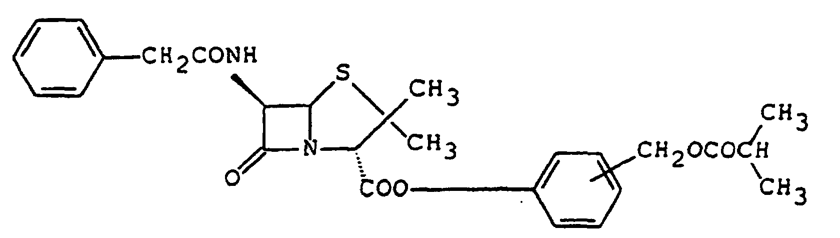 oder eines Salzes davon, welches Umsetzung einer Verbindung der Formel 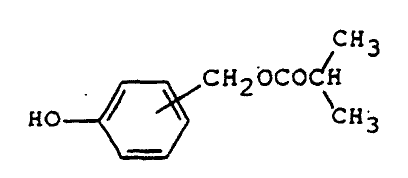 oder eines Salzes davon mit einer Verbindung der Formel 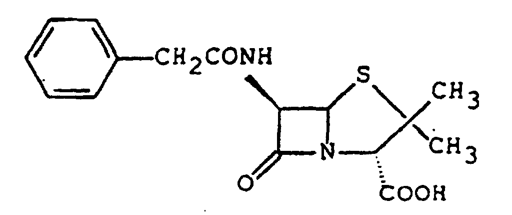 oder einem Salz davon umfasst.

2. Verfahren nach Anspruch 1, wobei die Reaktion in Gegenwart eines Kondensationsmittels durchgeführt wird.

3. Verfahren zur Herstellung eines Peniclllln-G-Phenylesters der Formel 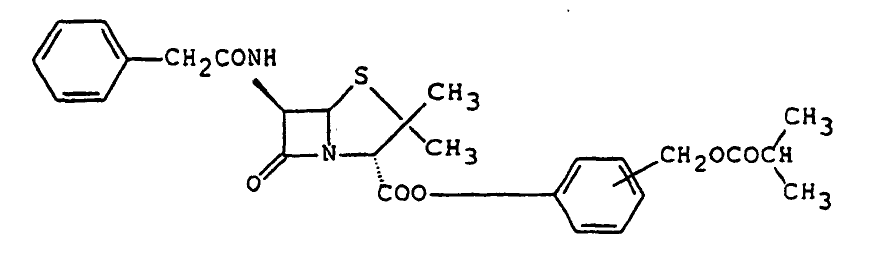 oder eines Salzes davon, welches Acylieren einer Verbindung der Formel 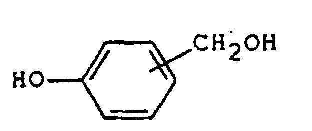 oder eines Salzes davon zur Herstellung einer Verbindung der Formel 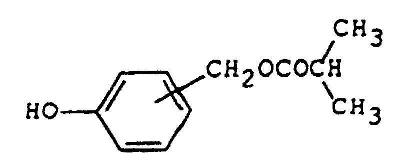 oder eines Salzes davon und Umsetzung desselben mit einer Verbindung der Formel 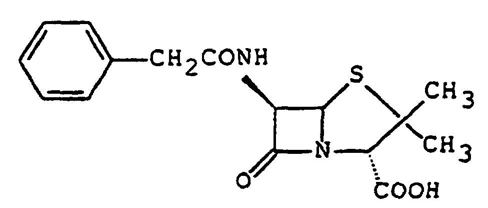 oder einem Salz davon umfosst.

4. Verfahren zur Herstellung eines Penicillin-G-Phenylesters der Formel 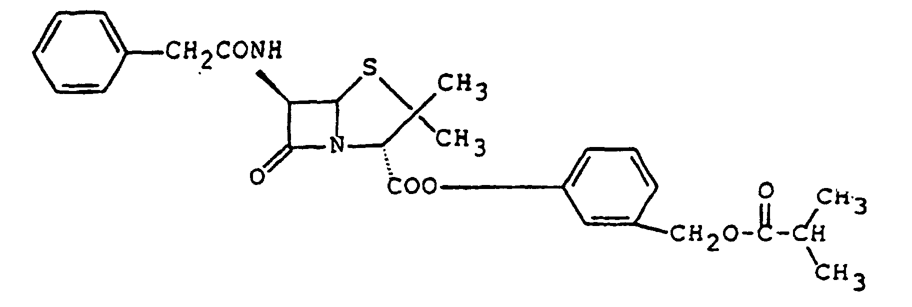 oder eines Salzes davon, welches Acylieren einer Verbindung der Formel 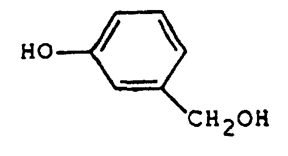 oder eines Salzes davon zur Herstellung einer Verbindung der Formel 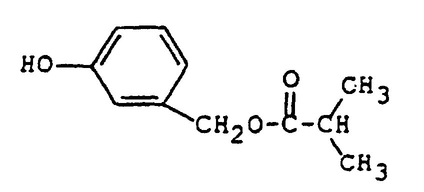 oder eines Salzes davon und Umsetzung derselben mit einer Verbindung der Formel 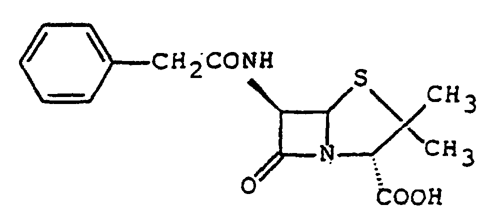 oder einem Salz davon umfasst.

5. Verbindung der Formel 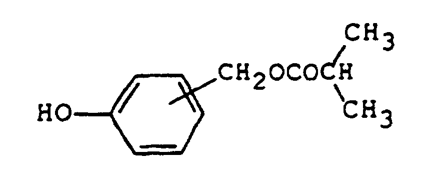 oder ein Salz davon.

6. Verbindung der Formel 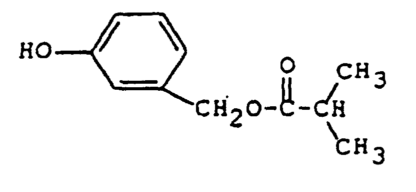 oder ein Salz davon.

## Revendications

1. Procédé de production d'un phényl-ester de pénicilline G de formule : 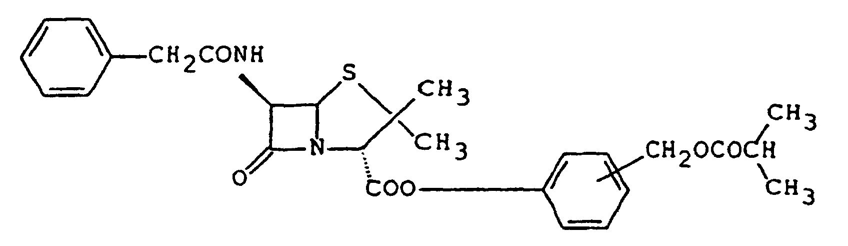 ou d'un sel de celui-ci, qui comprend la réaction d'un composé de formule : 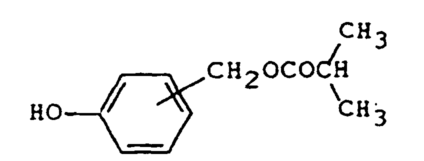 ou d'un sel de celui-ci, avec un composé de formule : 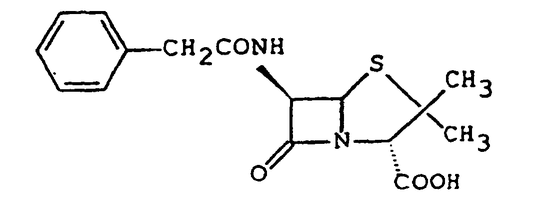 ou un sel de celui-ci.

2. Procédé selon la revendication 1, dans lequel la réaction est conduite en présence d'un agent de condensation.

3. Procédé de production d'un phényl-ester de pénicilline G de formule : 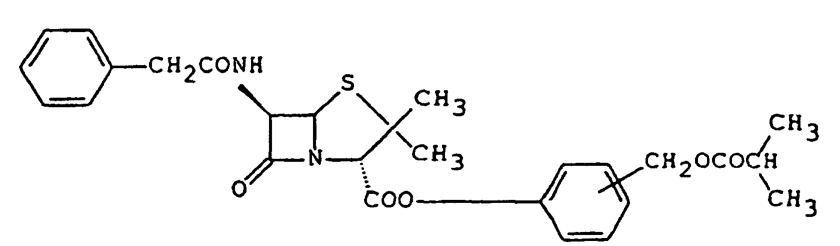 ou d'un sel de celui-ci, qui comprend l'acylation d'un composé de formule : 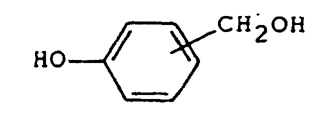 ou d'un sel de celui-ci, pour donner un composé de formule : 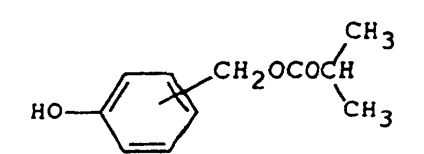 ou un sel de celui-ci, et la réaction de celui-ci avec un composé de formule : 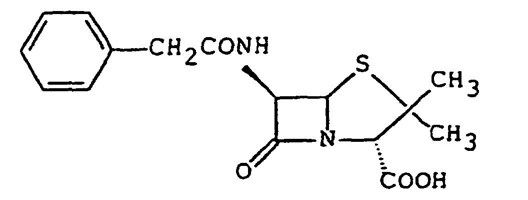 ou un sel de celui-ci.

4. Procédé de production d'un phényl-ester de pénicilline G de formule : 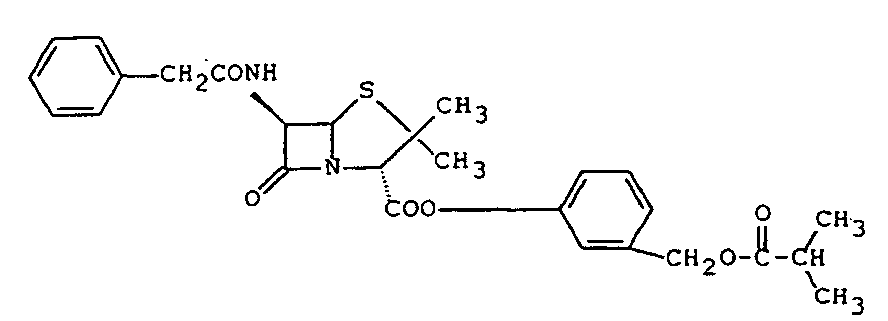 ou d'un sel de celui-ci, qui comprend l'acylation d'un composé de formule : 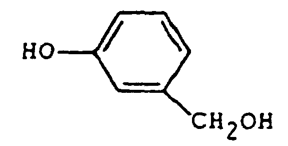 ou d'un sel de celui-ci, pour donner un composé de formule : 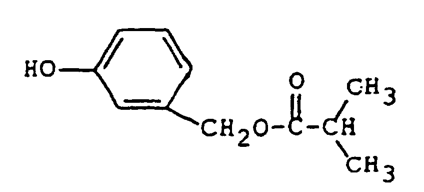 ou un sel de celui-ci, et la réaction de celui-ci avec un composé de formule : 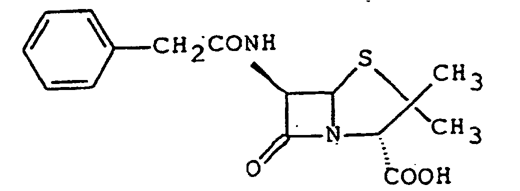 ou un sel de celui-ci.

5. Composé de formule : 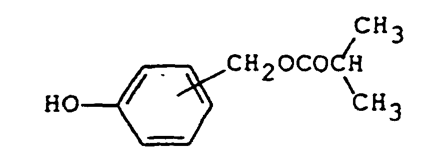 ou sel de celui-ci.

6. Composé de formule : 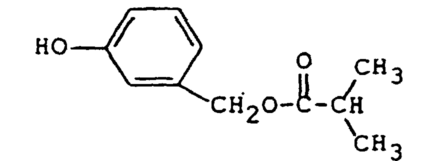 ou sel de celui-ci.
